# EUROPEAN PATENT SPECIFICATION

(11) **EP 0 419 235 B1**
(45) Date of publication and mention of the grant of the patent: **23.08.1995**
(21) Application number: 90310259.8
(22) Date of filing: 19.09.1990
(51) Int. Cl.: A61B 17/39

(54) **Loop electrode**
Schlingenelektrode
Electrode en forme de boucle

(30) Priority: 19.09.1989 GB 8921132
(43) Date of publication of application: 27.03.1991
(73) Proprietor: Rocket of London Limited, Watford, Hertfordshire WD2 4XX (GB)
(72) Inventor: Robson, Richard, Washington, Tyne & Wear (GB); Bartlett, Jonathan Easton, Morpeth, Northumberland, NE61 2EP (GB)
(74) Representative: Pattullo, Norman

(56) References cited:
- DE-A- 3 247 793
- DE-C- 848 233
- US-A- 3 807 404
- ELECTROMEDICA, vol. 41, no. 2, February 1983, pages 64-67; E. HOHMANN: "Das Sirotom ein Elektrochirurgiegerät für die Zahnheilkunde" Bürklin-Hauptkatalog 1982, pages L 31-32, H 28-29, H 58
- Bürklin-Hauptkatalog 1982, pages L 31-32, H 28-29, H 58

## Description

This invention relates to a medical instrument for use in diathermy.

In medicine, heat can be applied to the human body by high frequency currents in a process known as diathermy. Diathermic surgery involves the use of an electric current passing, for example, through a blade, forceps or thin wire in preference to a knife for cutting through tissue, and has the advantage of sealing the blood vessels at the cut and so reducing bleeding.

Diathermy is particularly appropriate for treatment of cervical disease. Iodine is used to stain the tissue of the cervix and the malignant cells show up as white patches. These cells can be excised by carrying out a biopsy using a diathermy loop, in the form of a re-usable hand-held instrument.

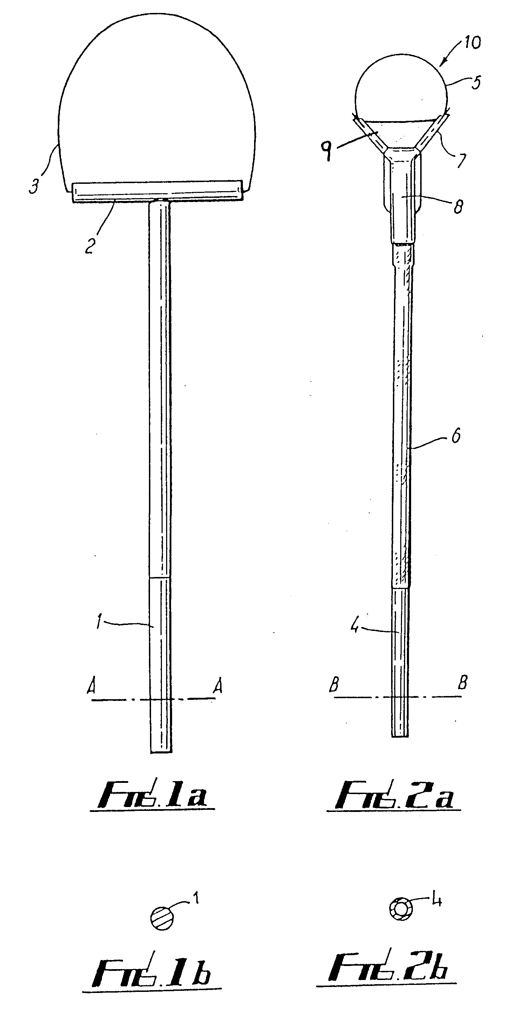

Figs. 1a and 1b show front and sectional views respectively of a typical diathermal instrument of the prior art. A solid metal rod 1 is welded to a metal tube 2 to form a T-junction. Wire 3 is looped from one end of the tube 2 to the other and the tube ends are stamped down around the wire ends to give the end product. Fig 1b shows a cross-section on line "A-A" of Fig 1a.

The instrument has disadvantages in that the wire may break at its connection to the tube and in order to provide sufficient mechanical strength the wire used is generally at least 0.25mm (0.010 inches) thick. However, it would be desirable to use thinner wire as such wire produces a more effective cutting action. Also, the welding point makes the production of the instrument costly.

German Patent DE-C-848233 discloses a medical instrument which comprises a tube having a cutting member extending from one end thereof, the cutting member describing a route passing through more than one plane.

US-A-3807404 discloses a probe unit having a disposable probe tip comprising a resilient wire which deforms upon insertion into the probe tip to engage with slots in the probe tip.

According to the present invention there is provided a method of making a medical instrument for use in diathermy, the method comprising providing a cutting member and a support member, both of the members being electrically conducting, and inserting two ends of the cutting member into one end of the support member, characterised in that when the two ends of the cutting member are inserted into the end of the support member, the end of the support member is deformed to secure the ends of the cutting member and to make an electrical connection between the members, and wherein insulating means covers a portion of the support member and forms a web spanning an area between the two ends of the cutting member.

Typically, the support member may be a metal tube which provides a handle for the instrument.

Typically, the cutting member is a wire, as it presents the same cutting surface to the tissue, no matter how it is manipulated. Preferably, the cutting member is less than 0.25mm (0.010 inches) thick, to give a more effective cutting action and most preferably is 0.15mm (0.006 inches) thick as this gives the best combination of mechanical strength and cutting action.

The insulating means may include sleeves covering the cutting member adjacent the point where it is received by the support member, and may also include a sheath which covers a length of the support member and is shrink-fitted onto the support member

The insulating means may be made from plastics material, and may also comprise a plastics (eg nylon) moulding which overlies the sleeves and joins the ends of the sleeves to the sheath.

An embodiment of the invention will now be described with reference to the drawings in which:
Fig. 2a is a front view of an embodiment of a medical instrument for use in diathermy of the present invention; and
Fig. 2b is a cross-section on B-B of Fig. 2.

A medical instrument 10 for use in diathermy (Fig. 2) comprises a support member in the form of a metal tube 4 and a cutting member in the form of a wire 5. Both ends of the wire 5 are received by the tube 4 and sections of the wire 5 adjacent the tube 4 are covered in plastic shrink-fitted sleeves 7. A plastic shrink-fitted sheath 6 covers part of the tube 4. The sleeves 7 are in turn enclosed in a nylon moulding 8, which covers the connection between the wire 5 and the tube 4 and completes the insulation so that it is continuous.

The instrument 10 has a moulded nylon section 8 which extends along the plastic sleeves 7 of the wire 5 to form a web 9.

This instrument is made by taking a stainless steel wire 0.15mm thick, threading a shrink-plastics sleeve on to each end, whilst leaving the ends themselves clear of the sleeve, inserting the ends of the wire into one end of a stainless steel tube 2.4 mm external diameter (13G) x 100mm long and flattening the tube so that the ends of the wire are trapped and an electrical connection is made. A moulding of low density polyethylene is made between the sleeves 7 and around the junction between the wires and tube which contacts the plastics sleeves and so the wire itself does not touch the wall of the mould. The wire loop may have a diameter of 15, 20 or 25mm depending on requirements.

In use, the instrument is attached to an electrical source, the wire heats up and is used to cut away tissue as required. The web 9 acts as a guide when using the instrument in that it limits the depth to which the instrument can cut.

The construction of the diathermal instrument of the present embodiment confers many advantages upon it. The instrument can be made at a lower cost than a re-usable instrument and is more effective as it has a steel wire almost half the diameter of the wire used in the prior art. The instrument is thus rendered more precise and effective than the conventional instruments.

Because the present invention is less expensive it may be provided as a disposable instrument which eliminates the problem of transfer of infection from inadequately-cleaned instruments.

Modifications and improvements may be incorporated without departing from the scope of the invention.

## Claims

1. A method of making a medical instrument for use in diathermy, the method comprising providing a cutting member (5) and a support member (4) both of the members (4, 5) being electrically conducting, and inserting two ends of the cutting member (5) into one end of the support member (4), characterised in that when the two ends of the cutting member (5) are inserted into the end of the support member (4), the end of the support member (4) is deformed to secure the ends of the cutting member (5) and to make an electrical connection between the members (4, 5), and wherein insulating means (6, 7, 8) are provided covering a portion of the support member (4) and forming a web (9) spanning an area between the two ends of the cutting member (5).

2. A method as claimed in Claim 1, characterised in that the support member (4) is tubular.

3. A method as claimed in Claim 1 or 2, characterized in that the cutting member (5) is a wire.

4. A method as claimed in Claim 1, 2 or 3 characterised in that the cutting member (5) is less than 0.25mm (0.01 inches) thick.

5. A method as claimed in Claim 4 characterized in that the cutting member (5) is substantially 0.15mm (0.006 inches) thick.

## Patentansprüche

1. Verfahren zum Herstellen eines medizinischen Instrumentes zur Verwendung in der Diathermie, wobei das Verfahren aus der Bereitstellung eines Schneidkörpers (5) und eines Stützkörpers (4) besteht, wobei die beiden Körper (4, 5) elektrisch leitend sind, und der Einfügung von zwei Enden des Schneidkörpers (5) in ein Ende des Stützkörpers (4), dadurch gekennzeichnet, daß wenn die beiden Enden des Schneidkörpers (5) in das Ende des Stützkörpers (4) eingefügt werden, das Ende des Stützkörpers (4) zur Sicherung der Enden des Schneidkörpers (5) und zur Herstellung einer elektrischen Verbindung zwischen den Körpern (4, 5) deformiert wird, wobei Isoliermittel (6, 7, 8) vorgesehen sind, die einen Teil des Stützkörpers (4) abdecken und einen Steg (9) bilden, der einen Bereich zwischen den zwei Enden des Schneidkörpers (5) überspannt.

2. Verfahren nach Anspruch 1, dadurch gekennzeichnet, daß der Stützkörper (4) rohrförmig ist.

3. Verfahren nach Anspruch 1 oder 2, dadurch gekennzeichnet, daß der Schneidkörper (5) ein Draht ist.

4. Verfahren nach Anspruch 1, 2 oder 3, dadurch gekennzeichnet, daß der Schneidkörper (5) weniger als 0.25 mm (0.01 inches) dick ist.

5. Verfahren nach Anspruch 4, dadurch gekennzeichnet, daß der Schneidkörper (5) im wesentlichen 0.15 mm (0.006 inches) dick ist.

## Revendications

1. Procédé de fabrication d'un instrument médical à usage en diathermie, le procédé comprenant le fait de prévoir un élément coupant (5) et un élément support (4), l'un et l'autre des éléments (4, 5) étant conducteurs de l'électricité, et le fait d'insérer deux extrémités de l'élément coupant (5) dans une extrémité de l'élément support (4), caractérisé en ce que lorsque les deux extrémités de l'élément coupant (5) sont insérées dans l'extrémité de l'élément support (4), l'extrémité de l'élément support (4) est déformée pour fixer les extrémités de l'élément coupant (5) et pour assurer une connexion électrique entre les éléments (4, 5), et dans lequel des moyens isolants (6, 7, 8) sont prévus pour couvrir une portion de l'élément support (4) et former un côté plat (9) traversant un espace entre les deux extrémités de l'élément coupant (5).

2. Procédé selon la revendication 1, caractérisé en ce que l'élément support (4) est tubulaire.

3. Procédé selon l'une des revendications 1 ou 2, caractérisé en ce rue l'élément coupant (5) est un fil métallique.

4. Procédé selon les revendications 1, 2 ou 3, caractérisé en ce rue l'élément coupant (5) a une épaisseur inférieure à 0,25 mm (0,01 pouces).

5. Procédé selon la revendication 4, caractérisé en ce que l'élément coupant (5) a une épaisseur sensiblement de 0,15 mm (0,006 pouces).
